# EUROPÄISCHE PATENTSCHRIFT

(11) **EP 0 712 610 B1**
(45) Veröffentlichungstag und Bekanntmachung des Hinweises auf die Patenterteilung: **27.05.1998**
(21) Anmeldenummer: 95118006.6
(22) Anmeldetag: 15.11.1995
(51) Int. Cl.: A61B 19/08, B29C 63/22, A61B 6/00

(54) **Folienabdeckung für medizinische Geräte**
Foil cover for medical devices
Enveloppe pour dispositifs médicaux

(30) Priorität: 15.11.1994 DE 4440806
(43) Veröffentlichungstag der Anmeldung: 22.05.1996
(73) Patentinhaber: Udo Heisig GmbH, 85609 Aschheim-Dornach (DE)
(72) Erfinder: Heisig, Ursula, 87521 München (DE)
(74) Vertreter: Feldkamp, Rainer, Dipl.-Ing.

(56) Entgegenhaltungen:
- EP-A- 0 140 858
- DE-A- 3 913 617
- US-A- 5 133 097

## Beschreibung

Die Erfindung bezieht sich auf eine Folienabdeckung für medizinische Geräte der im Oberbegriff des Anspruchs 1 genannten Art sowie auf ein Verfahren zur Herstellung einer derartigen Abdeckung.

Bei vielen medizinischen Geräten ist es erforderlich, auf den Geräteteilen eine sterile Abdeckung anzubringen, um die Übertragung von Keimen zu verhindern. Bei vielen medizinischen Geräten ergibt sich jedoch das Problem, daß hierbei zumindestens drei Seiten eines Geräteteils abgedeckt werden müssen und diese Geräteteile eine beispielsweise in ihrer Längsrichtung gekrümmte Form aufweisen, wie beispielsweise die sogenannten C-Bögen von Bestrahlungs- oder Durchleuchtungsgeräten.

Aus der DE 39 13 617-A-1 ist eine Vliesstoff-Sterilabdeckung für Röntgen-Bildverstärker-Einheiten gemäß der Oberbegriffe der Ansprüche 1 und 6 bekannt, die unter anderem zur Abdeckung eines C-Bogens einer solchen Einheit vorgesehen ist. Wie dies inbesondere den Fig. 14 und 15 dieser Druckschrift zu entnehmen ist, besteht der zum Abdecken des C-Bogens vorgesehene Teil der Abdeckung aus einer einzigen rechteckigen Materialbahn, die derart gefaltet wird, daß sich ein U-förmiger Querschnitt ergibt. Der der Innenseite des C-Bogens zugeordnete Materialabschnitt der Abdeckung muß wesentlich länger als die Innenoberfläche des C-Bogens sein, um ein Umschlagen der Seitenteile der Abdeckung um diesen C-Bogen zu ermöglichen.

Die Herstellung von Folienabdeckungen für derartige C-förmig gekrümmte oder andere gekrümmte Geräteteile ist sehr kompliziert, da diese Abdeckungen U-Form im Querschnitt haben müssen und andererseits auf der Innenseite eines gekrümmten Trägers anliegen und gleichzeitig dessen beide Seitenflächen bedecken müssen. Die beiden Seitenteile der Folienabdeckung haben damit die Form eines Kreisringsegmentes, und das Ausstanzen eines derartigen Kreisringsegmentes aus vollflächigem Folienmaterial (interner Stand der Technik) bedingt große Abfallmengen an Folie. Andererseits ist die genannte Form der Folienabdeckung erforderlich, um ein einwandfreies Anliegen dieser Folienabdeckung an einem gekrümmten Geräteteil sicherzustellen.

Der Erfindung liegt die Aufgabe zugrunde, eine Folienabdeckung der eingangs genannten Art sowie ein Verfahren zu deren Herstellung zu schaffen, das eine kostengünstige Herstellung mit geringen Abfallmengen an Folienmaterial ermöglicht.

Diese Aufgabe wird durch die in den Patentansprüchen 1 und 6 angegebenen Merkmale gelöst.

Vorteilhafte Ausgestaltungen und Weiterbildungen der Erfindung ergeben sich aus den Unteransprüchen.

Bei dem erfindungsgemäßen Verfahren werden für die Herstellung der Abdeckung für den gekrümmten Geräteteil drei geradlinige Folienstreifenabschnitte verwendet, wobei die die Seitenabschnitte bildenden Streifenabschnitte an ihrer mit dem Mittelabschnitt zu verbindenden Kante vor dem Verbinden gekräuselt und erst danach mit dem Mittelabschnitt, beispielsweise durch Verschweißen, verbunden werden.

Dieses Kräuseln kann entweder durch Anschweißen oder andere Befestigung eines elastisch gedehnten fadenförmigen Elementes, beispielsweise eines Gummibandes, an einer Kante der noch gestreckten Seitenabschnitte und nachfolgenes elastisches Zusammenziehen dieses fadenförmigen Elementes erfolgen, oder es werden Kräuselwalzen verwendet, die der für die Verbindung mit dem Mittelabschnitt vorgesehenen Kante der seitenabschnitte eine gekräuselte Form erteilen.

Ein Ausführungsbeispiel der Erfindung wird im folgenden anhand der Zeichnungen näher erläutert.

In der Zeichnung zeigen:
Fig. 1 eine vereinfachte schematische Darstellung einer Abdeckung, die einen C-Bogen eines medizinischen Gerätes abdeckt,
Fig. 2 eine Darstellung der einzelnen Teile der Abdeckung in auseinandergezogener Form.

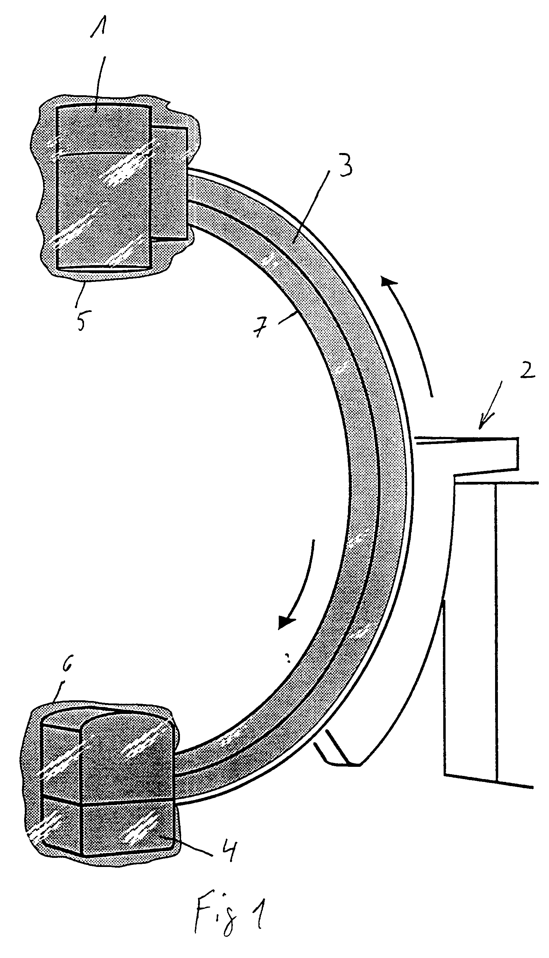

In Fig. 1 ist ein medizinisches Gerät dargestellt, das einen nur schematisch gezeigten Ständer 2 und einen daran befestigten, gegenüber dem Ständer auf einer Kreisbahn verschiebbaren Bogenteil 3 aufweist, der im folgenden als C-Bogen bezeichnet wird. An den Enden dieses C-Bogens 3 sind beispielsweise eine Strahlungsquelle 1 und ein Strahlungsempfänger 4 befestigt. Sowohl die Strahlungsquelle 1 als auch der Strahlungsempfänger 4 und der C-Bogen müssen im Gebrauch durch eine sterile Abdeckung abgedeckt werden.

Die Abdeckung der Strahlungsquelle 1 bzw. des Strahlungsempfängers 4 erfolgt mit Hilfe von im wesentlichen beutelförmigen Folienteilen 5 bzw. 6, die in üblicher Art ausgebildet sein können.

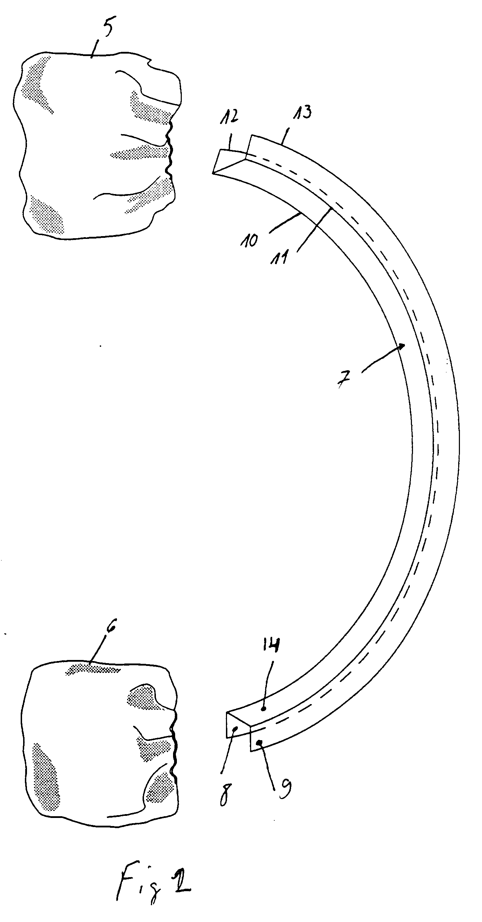

Die Abdeckung 7 des C-Bogens 3 ist jedoch relativ kompliziert, da diese Abdeckung 7 im Querschnitt eine U-Form aufweist und gekrümmte Seitenabschnitte 8, 9 (Fig. 2) nach Art eines Kreisringsegmentes aufweist.

Es ist selbstverständlich möglich, diese Seitenabschnitte 8, 9 der Abdeckung 7 durch Ausstanzen aus einem vollflächigen Material mit der vorgegebenen Kreisringsegmentform herzustellen, doch ergeben sich hierbei erhebliche Materialabfälle. Der Mittelabschnitt 14 der Abdeckung 7 weist demgegenüber eine unkomplizierte langgestreckte Streifenform auf.

Bei dem erfindungsgemäßen Verfahren werden die Seitenabschnitte 8, 9 als geradlinige Streifen aus Folienmaterial hergestellt, wobei an ihrer Innenkante 10, 11 vor dem Verschweißen mit dem Mittelabschnitt ein elastisch gedehntes fadenförmiges Element angeschweißt oder auf andere Weise befestigt wird, das bestrebt ist, sich nach der Freigabe zusammenzuziehen, so daß die die Innenkanten 10, 11 der seitenabschnitte 8, 9 bildenden Kanten gerafft bzw. gefältelt werden, während die Außenkanten 12, 13 ihre Länge beibehalten, so daß sich ein kreisringsegmentförmiges Aussehen der Seitenteile 8, 9 ergibt, die dann mit ihren Innenkanten 10, 11 mit dem Mittelabschnitt 14 verschweißt werden können, wobei die Fältelung flachgepreßt und mit verschweißt wird.

Bei einer anderen Ausführungsform werden die genannten Innenkanten der Seitenabschnitte nicht durch ein daran befestigtes elastisches fadenförmiges Element, sondern durch Hindurchleiten durch Kräuselwalzen gekräuselt.

Durch dieses Verfahren ergibt sich eine Folienabdeckung für kreisbogenförmig oder auf andere Weise gekrümmte Träger von medizinischen Geräten, die in sehr kostengünstiger Weise ohne wesentliche Abfallmengen herstellbar ist.

Weiterhin legt sich diese Abdeckung 7 aufgrund ihrer exakten Form sehr genau beispielsweise an den C-Bogen 3 nach Fig. 1 an, und kann an diesem beispielsweise durch doppeltklebendes Klebematerial oder Klammern befestigt werden.

Gemäß einer abgeänderten Ausführungsform des Verfahrens bzw. der Vorrichtung ist es weiterhin möglich, drei eine gleiche Länge aufweisende Folienstreifen zu verwenden, wobei die an der einen Seitenkante der seitlichen Folienabschnitte befestigten fadenförmigen Elemente bis zum Abschluß des Verschweißens der seitlichen Folienabschnitte mit dem mittleren Folienabschnitt im gedehnten Zustand gehalten werden. In diesem Fall ist es weiterhin möglich, diese fadenförmigen elastischen Elemente an den Seitenkanten des mittleren Folienabschnittes zu befestigen oder sie beim Verschweißen der seitlichen Folienabschnitte mit dem mittleren Folienabschnitt in die Schweißnaht zwischen diesen Abschnitten mit einzuschweißen. Hierbei ergibt sich jedoch nicht nur eine Kräuselung der Innenkanten der seitlichen Folienabschnitte sondern auch des mittleren Folienabschnittes insgesamt.

## Patentansprüche

1. Folienabdeckung für medizinische Geräte, die steril abzudeckende Geräteteile (3) aufweisen, die eine erhebliche Länge und eine in ihrer Längsrichtung gekrümmte Form aufweisen und eine bezogen auf die Krümmung innen liegende Innenfläche und zwei diese begrenzende Seitenflächen mit beispielsweise kreisringsegmentförmiger Form aufweisen,
dadurch gekennzeichnet, daß die Folienabdeckung (7) einen an die Abmessungen der Innenfläche des Geräteteils (3) angepaßten streifenförmigen Mittelabschnitt (14) und zwei Seitenabschnitte (8,9) mit einer an die Krümmung der Seitenflächen des Geräteteils angepaßten Form aufweist, und daß die aus einem gerade Seitenkanten aufweisenden Folienstreifen gebildeten Seitenabschnitte (8,9) eine Außenkante (12,13) und eine Innenkante (10,11) aufweisen, wobei diese Innenkanten (10,11) jeweils gekräuselt mit einer der Außenkanten des streifenförmigen Mittelabschnitts (14) verschweißt sind, so daß die Anpassung der Seitenabschnitte (8,9) an die Krümmung der Seitenflächen des Geräteteils erzielt wird.

2. Folienabdeckung nach Anspruch 1,
dadurch gekennzeichnet, daß die Innenkanten (10,11) der Seitenabschnitte (8,9) mit jeweils einem elastisch-gedehnten fadenförmigen Element verbunden sind, um nach Aufhebung der Dehnung des fadenförmigen Elementes die gekräuselte Verschweißung mit der Außenkante des streifenförmigen Mittelabschnittes (14) zu ermöglichen.

3. Folienabdeckung nach Anspruch 1 oder 2,
dadurch gekennzeichnet, daß die Folienabdeckung im Querschnitt im wesentlichen U-förmig ist.

4. Folienabdeckung nach Anspruch 2 oder 3,
dadurch gekennzeichnet, daß das dehnbare fadenförmige Element mit der Innenkante des jeweiligen Folienstreifens verschweißt ist.

5. Folienabdeckung nach einem der Ansprüche 2 bis 4,
dadurch gekennzeichnet, daß das fadenförmige Element ein Gummiband ist.

6. Verfahren zur Herstellung einer Folienabdeckung (7) für medizinische Geräte, die steril abzudeckende Geräteteile (3) aufweisen, die eine erhebliche Länge und eine in ihrer Längsrichtung gekrümmte Form aufweisen,
dadurch gekennzeichnet, daß die Folienabdeckung (7) aus drei eine gerade Längskante aufweisenden Folienabstreifen hergestellt wird, von denen ein erster einen Mittelabschnitt (14) und zwei weitere jeweilige mit den Seitenkanten des Mittelabschnittes verbundene Seitenabschnitte (8,9) bilden, daß die mit den Seitenkanten des Mittelabschnittes (14) zu verbindenden Kanten (10, 11) der Seitenabschnitte (8,9) vor der Verbindung mit dem Mittelabschnitt (14) gekräuselt werden.

7. Verfahren nach Anspruch 6,
dadurch gekennzeichnet, daß die Kräuselung dadurch erzielt wird, daß ein elastisches fadenförmiges Element im gedehnten Zustand mit der für die Verbindung mit dem Mittelabschnitt (14) vorgesehenen Innenkante (10,11) des jeweiligen Seitenabschnittes (8,9) verbunden und die Dehnung dann aufgehoben wird, um die Kräuselung dieser Innenkante (10,11) der Seitenabschnitte (8,9) zu erzielen.

8. Verfahren nach Anspruch 6,
dadurch gekennzeichnet, daß die zur Verbindung mit dem Mittelabschnitt (14) vorgesehene Innenkante (10,11) der Seitenabschnitte (8,9) durch Hindurchführen durch Kräuselwalzen gekräuselt wird.

9. Verfahren nach Anspruch 6,
dadurch gekennzeichnet, daß die Dehnung der elastischen fadenförmigen Elemente erst nach dem Verbinden des Mittelabschnittes (14) mit den Seitenabschnitten (8,9) aufgehoben wird.

10. Verfahren nach Anspruch 9,
dadurch gekennzeichnet, daß die elastischen fadenförmigen Elemente im gedehnten Zustand in eine Schweißnaht zwischen dem Mittelabschnitt (14) und den Seitenabschnitten (8,9) eingeschweißt werden.

## Claims

1. Foil covering for medicinal appliances, which have parts [3] to be covered in a sterile condition and which have a considerable length and a curved shape in their longitudinal direction, and an inner face lying on the inside of the curve and two side faces defining the inner face with for example the shape of a segment of a circle, characterised in that the foil covering [7] has a strip-like middle section [14] matching the dimensions of the inner face of the appliance part [3] and two side sections [8, 9] with a shape matching the curvature of the side faces of the appliance part, and in that the side sections [8, 9] consisting of foil strips having straight side edges have an outer edge [12, 13] and an inner edge [10, 11], these inner edges [10, 11] each being welded in a curved state to an outer edge of the strip-like middle section [14], so that the side sections [8, 9] can be made to match the curvature of the side faces of the appliance part.

2. Foil covering according to claim 1, characterised in that the inner edges [10, 11] of the side sections [8, 9] are each connected to an elastically stretched thread-like element in order, after release of the thread-like element, to permit curled welding to the outer edge of the strip like middle section [14].

3. Foil covering according to claim 1 or 2, characterised in that the foil covering is U-shaped in cross-section.

4. Foil covering according to claim 2 or 3, characterised in that the stretchable thread-like element is welded to the inner edge of the respective foil strip.

5. Foil covering according to one of claims 2 to 4, characterised in that the thread-like element is a rubber band.

6. Method of producing a foil covering [7] for medicinal appliances, which have parts [3] to be covered in a sterile condition, which have a considerable length and a curved shape in the longitudinal direction, characterised in that the foil covering [7] consists of three foil strips having a straight longitudinal edge, of which a first forms a middle section [14] and two further strips each form side sections [8, 9] connected to the side edges of the middle section, and in that the edges [10, 11], of the side sections [8, 9], to be connected to the side edges of the middle section [14] are curled before connection to the middle section [14].

7. Method according to claim 6, characterised in that the curling is achieved by joining an elastic thread-like element in the stretched state to the inner edge [10, 11] of each side section [8, 9] provided for connection to the middle section [14] and stretching is then discontinued in order to achieve the curling of this inner edge [10, 11] of the side sections [8, 9].

8. Method according to claim 6, characterised in that the inner edge [10, 11] provided for joining to the middle section [14] is curled by passing through curling rollers.

9. Method according to claim 6, characterised in that the stretching of the elastic thread-like elements is not discontinued till after joining of the middle section [14] to the side sections [8, 9].

10. Method according to claim 9, characterised in that the elastic thread-like elements are welded in the stretched state into a weld seam between the middle section [14] and the side sections [8, 9].

## Revendications

1. Enveloppe de protection en film destinée à des appareils médicaux comportant des éléments d'appareil (3) à protéger de manière stérile, qui présentent une grande longueur et une forme incurvée dans leur direction longitudinale et qui comportent une surface intérieure, située a l'intérieur par rapport à la courbure, ainsi que deux surfaces latérales, par exemple ayant la forme de segments d'anneaux circulaires, qui délimitent ladite surface intérieure, caractérisée par le fait que l'enveloppe de protection en film (7) présente un panneau de milieu (14) en forme de bande adapté aux dimensions de la surface intérieure de l'élément d'appareil (3) et deux panneaux de côté (8, 9) avec une forme adaptée a la courbure des surfaces latérales de l'élément d'appareil et par le fait que les panneaux de côté (8, 9) formés d'une bande de film rectiligne avec des bords latéraux présentent un bord extérieur (12, 13) et un bord intérieur (10, 11), lesdits bords intérieurs (10, 11) froncés étant soudés a l'un des bords extérieurs du panneau de milieu (14) en forme de bande, de telle sorte que les panneaux de côté (8, 9) s'adaptent a la courbure des surface latérales de l'élément d'appareil.

2. Enveloppe de protection en film selon la revendication 1, caractérisée par le fait que les bords intérieurs (10, 11) des panneaux de côté (8, 9) sont liés chaque fois à un élément en forme de fil étiré élastiquement pour permettre après le relâchement de l'élément en forme de fil la soudure froncée avec le bord extérieur du panneau de milieu en forme de bande.

3. Enveloppe de protection en film selon la revendication 1 ou 2, caractérisée par le fait que l'enveloppe, en section transversale, a essentiellement une forme de U.

4. Enveloppe de protection en film selon la revendication 2 ou 3, caractérisée par le fait que l'élément extensible en forme de fil est soudé au bord intérieur de la bande de film concernée.

5. Enveloppe de protection en film selon une des revendications 2 à 4, caractérisée par le fait que l'élément en forme de fil est une bande de caoutchouc.

6. Procédé de fabrication d'une enveloppe de protection en film (7) pour des appareils médicaux comportant des éléments d'appareil (3) qui doivent être protégées de manière stérile et qui présentent une longueur importante et une forme courbe dans le sens de leur longueur, caractérisé par le fait que l'enveloppe de protection en film (7) est fabriquée à partir de trois bandes de film qui présentent un bord longitudinal rectiligne, parmi lesquelles une première bande forme un panneau de milieu (14) et deux bandes supplémentaires forment chacune des panneaux de côté (8, 9) qui sont liés aux bords latéraux du panneau de milieu, par le fait que les bords (10, 11) des panneaux de côté (8, 9) à lier aux bords latéraux du panneau de milieu (14) sont froncés avant leur liaison avec ledit panneau de milieu (14).

7. Procédé selon la revendication 6, caractérisé par le fait que les fronces sont obtenues en liant un élément en forme de fil élastique à l'état étiré au bord intérieur (10, 11) du panneau de côté (8, 9) concerné prévu pour la liaison avec le panneau de milieu (14) et en relâchant la tension de l'élément en forme de fil pour obtenir le fronçage dudit bord intérieur (10, 11) du panneau de côté (8, 9).

8. Procédé selon la revendication 6, caractérisé par le fait que l'on fronce le bord intérieur (10, 11) des panneaux de côté (8, 9) prévu pour la liaison avec le panneau de milieu (14) par passage entre des cylindres fronceurs.

9. Procédé selon la revendication 6, caractérisé par le fait que l'on relâche la tension de l'élément en forme de fil élastique seulement après réalisation de la liaison entre le panneau de milieu (14) et les panneaux de côté (8, 9).

10. Procédé selon la revendication 9, caractérisé par le fait que les éléments en forme de fil élastiques sont fixés à l'état étiré dans un cordon de soudure entre le panneau de milieu (14) et les panneaux de côté (8, 9).
